# EUROPEAN PATENT APPLICATION

(11) **EP 1 844 698 A2**
(43) Date of publication of application: **17.10.2007**
(21) Application number: 07007354.9
(22) Date of filing: 10.04.2007
(51) Int. Cl.: A61B 1/12, A61B 19/00, A61L 2/16, A61L 2/18, A46B 5/06, B08B 9/04

(54) **Apparatus for washing and disinfecting endoscope and brush unit for washing ducts of endoscope**

(30) Priority: 10.04.2006 JP 2006108113
(71) Applicant: OLYMPUS MEDICAL SYSTEMS CORP., Shibuya-ku Tokyo 151-0072 (JP)
(72) Inventor: Suzuki, Eiri, Tokyo 151-072 (JP); Hasegawa, Hitoshi, Tokyo 151-072 (JP); Suzuki, Shintaro, Tokyo 151-072 (JP); Noguchi, Toshiaki, Tokyo 151-072 (JP); Kobayashi, Kenichi, Tokyo 151-072 (JP); Ogawa, Akihisa, Tokyo 151-072 (JP)
(74) Representative: von Hellfeld, Axel

(57) **Abstract**

An apparatus (1) for washing and disinfecting an endoscope (50) comprises a frame (2A), a brush unit (20), and a lid (3). The frame is equipped with a washing bath (4) accommodating therein the endoscope for washing and disinfection. The brush unit accommodates therein a brush (30) for washing a duct of the endoscope and is detachably loaded to the frame at a predetermined position thereof to allow the brush to be inserted into the duct. The lid is attached to the frame to selectively open and close and formed to cover the washing bath and the brush unit loaded to the fame when the frame is closed. Further, the apparatus comprises a reuse-disabling device (34, 39) disabling the brush unit from being reused in association with the opening and closing actions of the lid. The reuse-disabling device prevents reuse of the brush unit which is poor in its washing performance due to being used already.

## Description

### CROSS REFERENCE TO RELATED APPLICATION

The patent application related to and incorporates by reference Japanese Patent application No. 2006-108113 filed on April 10, 2006.

### Background of the Invention

### (The field of the Invention)

The present invention relates to an apparatus for washing and disinfecting used endoscopes and a brush unit, used with such a washing-disinfecting apparatus, for washing ducts of an endoscope.

An effective way for inspecting and treating the inside of cavities of an object being diagnosed is to use an endoscope. The endoscope is provided with an insertion tube to be inserted into a body cavity, and there are formed various endoscopic ducts (hereinafter, referred as endoscopic channels or more simply, channels) inside the insertion tube. The channels include a suction channel which also functions as a forceps channel. Thus, once being used, the insertion tube is polluted by humor, such as mucosa and blood, and filth attached to the outer surface thereof as well as the inside of the channels (i.e., ducts). It is therefore necessary to sufficiently wash and disinfect both the outer surface and the endoscopic channels of the insertion tube, every time the endoscope has been used.

To cope with such situations, an endoscope washing and disinfecting apparatus, which is able to wash the endoscope, is disclosed by Japanese Patent Laid-open Publication No. 2002-209847. This endoscope washing and disinfecting apparatus is equipped with a wire accommodating chamber in which a reel to take up a wire of a washing brush is accommodated. The washing brush is for washing endoscopic channels.

However, in the conventional endoscope washing and disinfecting apparatus described with the above publication, it is necessary to replace, with a new non-used washing brush, the used washing brush whose washing performance is lowered with new one. This replacement should be done by a user, whenever the washing brush is used, because its washing performance is obliged to be degraded due to the use. The replacement work involves unloading and loading the washing brushes, which is always troublesome work for the user.

In addition, the foregoing endoscope washing and disinfecting apparatus tends to involve human errors which result in failures in exchanging washing brushes. This means that there is always a possibility that the used washing brush of which washing performance is deteriorated is used again by mistake. Such a case, if it really happens, may lead to an insufficient washing finish of ducts of an endoscope due to the poor washing performance, even when the same washing procedures as those provided by the predetermined washing and disinfecting routine are carried out.

### Summary of the Invention

The present invention has been made in consideration of the foregoing conventional situations, and has an object to provide an endoscope washing and disinfecting apparatus and a washing brush unit, which are capable of not only saving labor for exchanging brushes but also steadily preventing a situation where a once-used, poor-washing-performance brush is used again by mistake, when a duct(s) (endoscopic channel(s)) of the used endoscope is subject to brush washing.

In order to accomplish the above object, the present invention provides, as one aspect, an endoscope washing and disinfecting apparatus (1) for washing and disinfecting an endoscope (50) having a duct. The apparatus comprises a frame (2A) equipped with a washing bath (4) accommodating therein the endoscope for washing and disinfection; a brush unit (20) accommodating therein a brush (30) for washing the duct of the endoscope and detachably loaded to the frame at a predetermined position thereof to allow the brush to be inserted into the duct; a lid (3) attached to the frame to selectively open and close and formed to cover the washing bath and the brush unit loaded to the fame when the frame is closed; and a reuse-disabling device (34, 39) disabling the brush unit from being reused in association with the opening and closing actions of the lid.

In addition, as another aspect, the present invention provides a brush unit detachably loaded to an apparatus for washing and disinfecting an endoscope. The brush unit comprises a brushing device (30), a driving member (22a, 22b), a casing (24), and a reuse-stopping member (26). The brushing device is equipped with a brush (31) washing a duct of the endoscope. The drive member (22a, 22b) drives the brushing device to run the brush in and along the duct of the endoscope. The casing (24) accommodates therein the brushing device and the drive member and is detachably loaded to the washing and disinfecting apparatus. Further, the reuse-stopping member (26) is arranged at the casing and stops the brush unit from being reused.

By the endoscope washing and disinfecting apparatus and the brush unit according to the present invention, ducts of an endoscope which has been used once can be washed by the brush in a sanitary and effective manner. Additionally, the brush unit is detachable to and from the apparatus, so that the unit can be replaced, unit by unit, by a new one. Thus work for replacing the units is easier. Further, the washing performance of the brush is reduced, once being used. This means that reusing the brush unit without replacing the brush with a new one will bring about a problem in providing a predetermined level of cleanness to ducts of an endoscope. The present invention can get rid of this problem.

That is, after finishing the washing work for the duct(s) of an endoscope, an operator opens the lid of the endoscope washing and disinfecting apparatus. This open of the lid will cause the previously used brush unit to be disabled from being reused (i.e., stopped from being used again). Hence, the same brush unit cannot be loaded to the endoscope washing and disinfecting apparatus again. Accordingly, it is surely avoidable to reuse, by mistake, the bush unit of which washing performance is degraded due to the previous usage.

### Brief Description of the drawings

In the accompanying drawings:
Fig. 1 is a perspective view showing the appearance of an endoscope washing and disinfecting apparatus according to an embodiment of the present invention, in which the apparatus with its top cover opened is illustrated;
Fig. 2 is a perspective view showing the endoscope washing and disinfecting apparatus of which top cover is closed;
Fig. 3 is a perspective view showing a washing brush unit employed in the embodiment;
Fig. 4 is a plan view of the washing brush unit;
Fig. 5 is a sectional view of the washing brush unit;
Fig. 6 is a sectional view showing a cassette mounting portion of the apparatus on which the washing brush unit is to be mounted;
Fig. 7 is a view illustrating how the washing brush unit is mounted onto the cassette mounting portion;
Fig. 8 is a view illustrating how the top cover is closed to the apparatus to enclose the washing brush unit;
Figs. 9 and 10 respectively showing how a cassette pressing member presses an exhaust-side connecting pipe;
Fig. 11 is a view how the top cover is opened and how part of the washing brush cassette is positively broken;
Fig. 12 outlines a flowchart executed by a control unit; and
Fig. 13 is a partial sectional view showing a modification for a positive deformation of the washing brush cassette.

### Detailed Description of the Preferred Embodiments

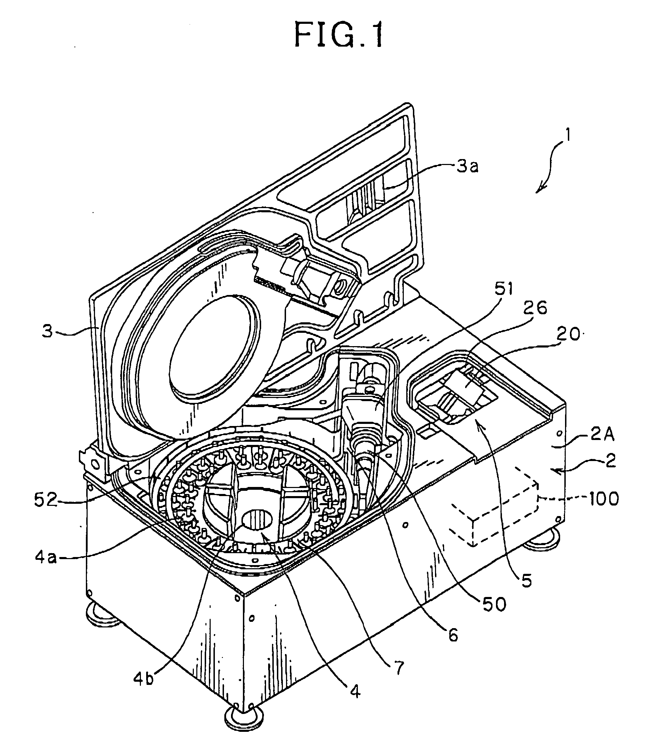

With reference to Figs. 1 - 12, an embodiment of an endoscope washing and disinfecting apparatus will now be described, which includes a description of a brush unit for washing a duct(s) of an endoscope. Hereinafter, such a brush unit is referred to as a "washing brush unit."

In Fig. 1, an endoscope washing and disinfecting apparatus 1 according to the present embodiment is shown. This endoscope washing and disinfecting apparatus 1 is equipped with a main body unit 2 and a top cover 3 serving as a lid which can be opened and closed with the top plane of the main body unit 2. The main body unit 2 is provided with a frame 2A serving as a casing, of which entire appearance is an approximately box-like shape. Inside the frame 2A, there are electric and mechanical facilities for washing and disinfecting endoscopes.

On the top portion of the frame 2A are provided a washing and disinfecting bath (hereinafter, simply referred as a washing bath) 4 and a mounting portion (hereinafter, referred as a cassette mounting portion) 5 at which a washing brush unit is mounted. An endoscope 50 to be washed and disinfected is accommodated within the washing bath 4.

The washing base 4 includes a manipulating-member accommodating bath 6 which agrees in shape with a manicuring member 51 of the endoscope 50 and an insertion-tube accommodating bath 7 which allows an insertion tube 52 of the endoscope 50 to be accommodated in a circular form therein. The insertion-tube accommodating bath 7 is provided with a plurality of support members 4a and a washing-case accommodating portion 4b. The support members 4a are used to support the circular-accommodated insertion tubes 52 so that the tubes 52 are mutually separated and located at predetermined intervals. The washing-case accommodating portion 4b, which is located at the center of the base 4, supports a not-shown washing case. This washing case accommodates therein various accessories, such as buttons and forceps taps, of the endoscope 50 and is used to wash and disinfect such accessories together with the endoscope 50 itself.

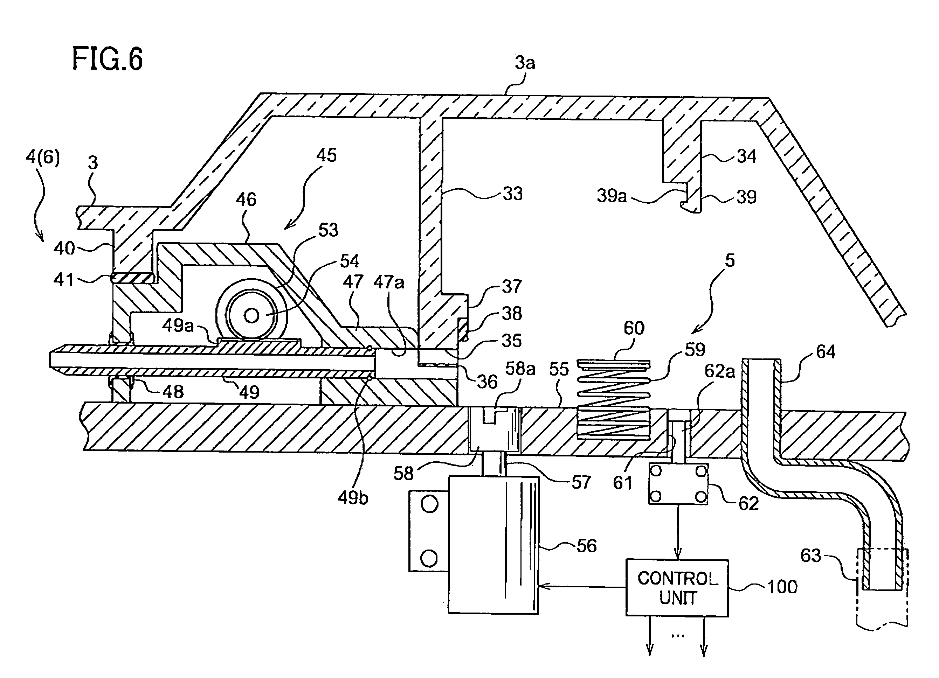

The cassette mounting portion 5 is formed close to the manipulating-member accommodating bath 6. In order to make a connection to or disconnection from a mouthpiece of a duct formed through the endoscope 50, the cassette mounting portion 5 is provided with a washing nozzle and a washing-nozzle connecting/disconnecting mechanism 45 (refer to Fig. 6), which will be detailed later. Simply, the washing nozzle is made to go back and forth from and toward the manipulating member 51. The washing-nozzle connecting/disconnecting mechanism 45 is placed to cause the washing nozzle to go backward and forward.

The washing brush unit according to the present embodiment is realized as a detachable-type of washing brush cassette 20 and mounted at the cassette mounting portion 5.

On the top cover 3 of the apparatus 1 is formed a cassette cover 3a, which is shaped into a recess when viewing it from the rear side of the top cover 3. This cassette cover 3a is formed to prevent the washing brush cassette 20 mounted at the cassette mounting portion 5 from touching the back of the top cover 3 which is closed. The top cover 3 is made of clear or translucent material, which allows users to observe the washing bath 4 and the washing brush cassette 20 through the closed top cover 3.

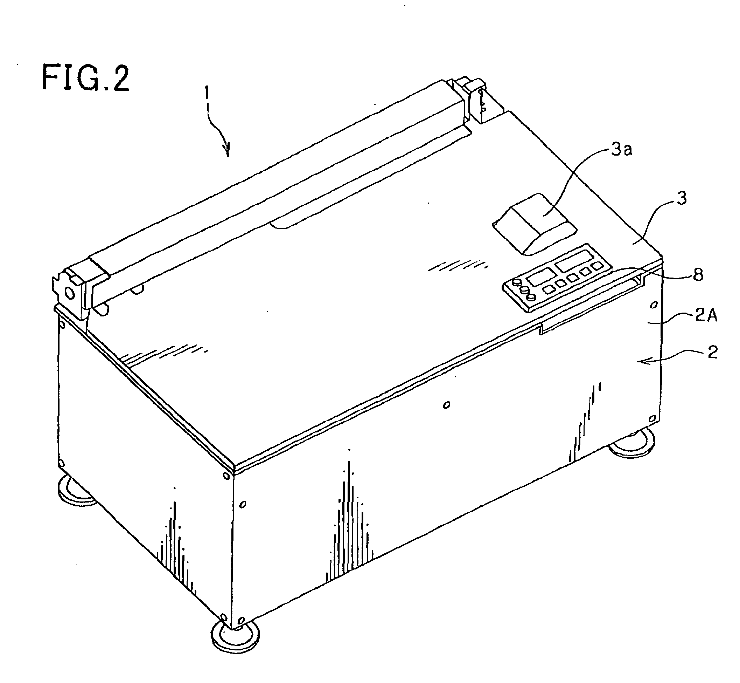

In addition, as shown in Fig. 2, an operation/display panel 8 is provided on the upper surface of the top cover 3. This panel 8 is provided various operation and display devices which are interactive with user's operations and massages to users. Such operations and massages include stat and stop operations, display for various steps, display of elapsed time, setting operations of washing and disinfecting steps.

In the endoscope washing and disinfecting apparatus 1, the frame 2 accommodates therein various mechanisms which allow washing liquid, disinfecting liquid, rinsing water, alcohol, and air to circulate into or through the washing bath 4 and cassette mounting portion 5 in various work steps of washing, disinfecting, alcohol-flushing, and dewatering. Such fluids circulated are for washing and disinfecting the endoscope 50. For accomplishing the circulation, a tubing network with electromagnetic valves, check valves and other devices and various devices such as pumps and compressors are incorporated in the frame 2A. In addition, a control unit 100 (refer to Fig. 1) is also incorporated in the main body unit 2 and is configured to drive the foregoing devices in accordance with programs assigned to the various washing and disinfecting steps.

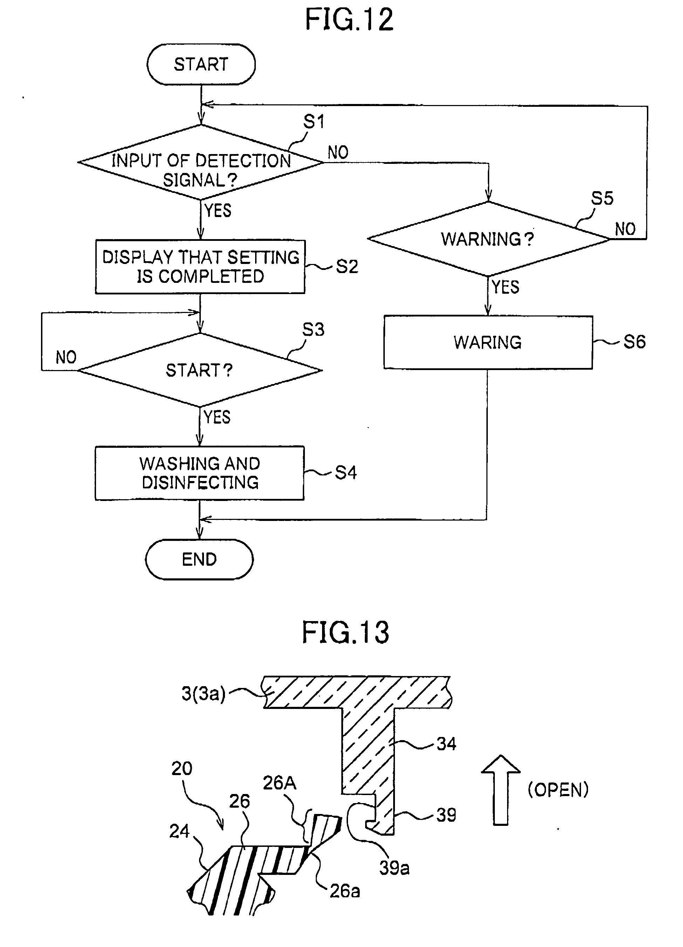

The control unit 100 is formed as a computer equipped with memories and a CPU (central processing unit). The memories store therein programs for the washing and disinfecting steps and a program for monitoring loaded states of a washing brush cassette 20 which will be explained later. The CPU is configured to execute such programs. Depending on the actions of the CPU, the control unit 100 executes the processing shown in Fig. 12 later explained.

The washing liquid, disinfecting liquid, and alcohol liquid are reserved in respective tanks placed in the frame 2A. Additionally, to this endoscope washing and disinfecting apparatus 1, the rinsing water is supplied from the hydrant through a not-shown hose connected with the frame 2A. The tap water is used to dilute the washing liquid and disinfecting liquid.

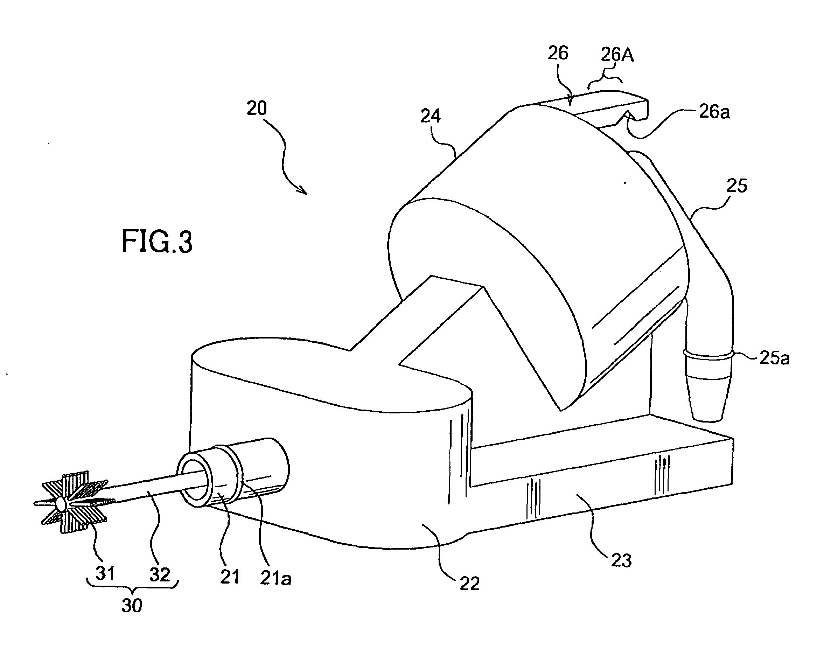

Referring to Figs. 3-5, the washing brush cassette 20 to be mounted on the cassette mounting portion 5 will now be detailed.

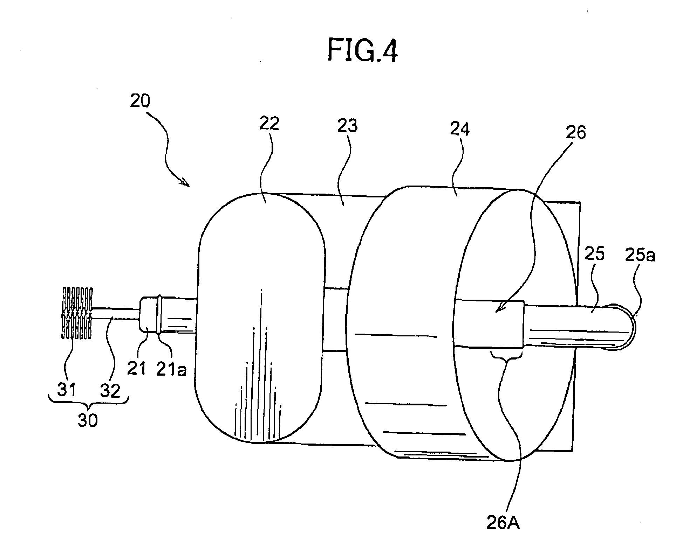

As shown in Figs. 3 and 4, the washing brush cassette 20 is provided with, as its main members, an exhaust-side connecting pipe 21, roller accommodating portion 22, base portion 23, washing-brush accommodating portion 24, and suction-side connecting pipe 25.

Of these members, the exhaust-side connecting pipe 21 is arranged to extend forward from at an approximately central part on the front of the roller accommodating portion 22. On the outer circumferential surface of this pipe 21, an O-ring 21a is attached placed to neighbor the opening thereof. This O-ring 21a provides water tightness to a gap between the outside and the inside of the cassette 20 mounted on the cassette mounting portion 5.

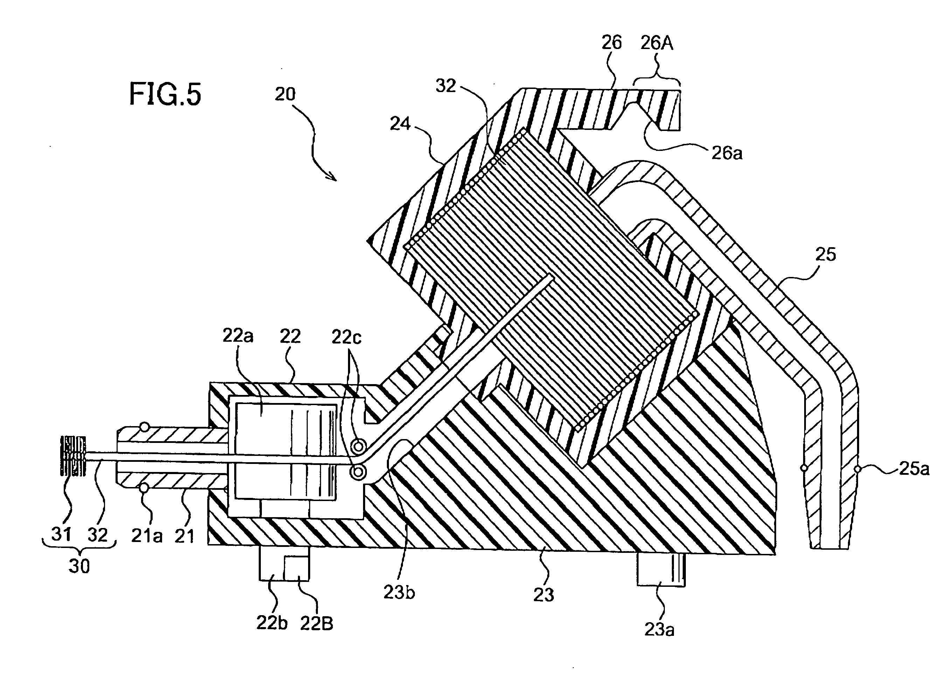

The roller accommodating portion 22 is formed to have a chamber communicating with the exhaust-side connecting pipe 21, as shown in Fig. 5, and formed into an ellipse shape, when viewed from a viewpoint thereabove, as shown in Fig. 4. In the roller accommodating portion 22, there are provided with a driving roller 22a and a not-shown driven roller. The driving roller 22a has a roller shaft 22b rotatably and air-tightly supported at a part of the bottom of the roller accommodating portion 22. The roller shaft 22b has an engaging portion 22B notched in a predetermined shape at the lower end thereof.

At a base-side position of the inner chamber of the roller accommodating portion 22, two stick-like brush guides 22c are arranged to extend along the width direction of the roller accommodating chamber 22. The brush guides 22c are arranged hold and pinch a shaft wire 32 of a washing brush 30 in the vertical direction of the chamber 22.

The base portion 23 has a lower surface on which two protrusions 23a are formed (in Fig. 5, only one protrusion is shown) and also has a communication path 23b communicating to the inner chamber of the roller accommodating portion 22.

The washing-brush accommodating portion 24, which is made of a plastic resin, for example, is a member formed into an approximate cylinder with an inner chamber therein and attached on the upper surface of the base portion 23 in an attitude tilting to the base portion 23. In the inner chamber of the washing-brush accommodating portion 24, the shaft wire 32 of the washing brush 30 is wound and accommodated. The inner chamber of this portion 24 is formed to communicate with a communicating path 23b formed in the base portion 23.

As well shown in Figs. 3 - 5, the upper surface of the washing-brush accommodating portion 24 is formed to have an arm-shaped protrusion 26 made of resin, for example. This arm-shaped protrusion 26 is formed as one unit with the accommodating portion 24 and formed to extend backward in a branch shape. The arm-shaped protrusion 26 has a tip 26A which serves as a portion to be broken, as will be explained later.

To be specific, in the present embodiment, the arm-shaped protrusion 26 is formed into an approximate rectangular-column member. This arm-shaped protrusion 26 has a lower surface, at a predetermined potion of which a recessed notch (groove) 26a is formed by cutting off part of the column member, as shown in Fig. 5. This notch 26a, which functions as a breaking groove, is formed to pass almost perpendicularly to the longitudinal direction of the arm-shaped protrusion 26. The position of depth of the notch 26a are designed in consideration of the strength of material of the protrusion 26 and a forth applied when opening the top cover 3 such that the tip 26A of the arm-shaped protrusion 26 is surely broken off at the notch 26a responsively to opening the top cover 3.

The suction-side connecting pipe 25 is elbowed, directed downward, and has both ends one of which is connected to a central part of a declined base-side surface of the washing-brush accommodating portion 24. This pipe 25 is made to communicate with an inner chamber of the washing-brush accommodating portion 24. An O-ring 25a is attached to the outer surface of this pipe 25 at a position close to the other end thereof.

Thus, in the washing brush cassette 20, both of the connecting pipes 21 and 25 provide with the cassette 20 with the openings. And the exhaust-side connecting pipe 21, the inner chamber of the roller accommodating portion 22, the communicating path 23b of the base portion 23, the inner chamber of the washing-brush accommodating portion 24, and the suction-side connecting pipe 25 are mutually communicated with each other to allow liquid to pass through.

The washing brush 30, which is incorporated within the washing-brush accommodating portion 24, is provided with a brush portion 31 and the foregoing shaft wire 32 at a tip of which the brush portion 31 is arranged. The brush portion 31 has resin-made brushing hairs. In the present embodiment, the wire shaft 32 is made of resin or stainless steel. The shaft wire 32 passes through the communicating path 23b of the base member 23 and pressed between mutually faced rolling surfaces of both of the driving roller 22a and the driven roller (not shown) within the roller accommodating portion 22. The washing brush cassette 20 is mounted such that the bush portion 31 comes out of the exhaust-side connecting pipe 21.

The thus-constructed washing brush cassette 20 is detachably mounted on the cassette mounting portion 5, at which the washing-nozzle connecting/disconnecting mechanism 45 is arranged to be exposed to the outside at the edge of the endoscope washing and disinfecting apparatus 1.

Referring to Fig. 6, descriptions will be given to the constructions of the cassette cover 3a of the top cover 3, the washing-nozzle connecting/disconnecting mechanism 45, and the cassette mounting portion 5.

As shown in Fig. 6, the cassette cover 3a formed as part of the top cover 3 comprises a cassette pressing member 33 and a portion-being-broken latch member 34. Both members 33 and 34 are formed into almost rod-like members having predetermined lengths, respectively, and are integrally formed with the cassette cover 3a so that the members 33 and 34 extend almost perpendicularly from the cover 3a. The cassette pressing member 33 serves as a first securing member extending downward from the back (recessed surface, as shown in Fig. 6) of the cassette cover 3a. On the other hand, the portion-being-broken latch member 34 serves as a second securing member also extending downward from the back of the cassette cover 3a. This engaging member 34 is located farer than the member 33.

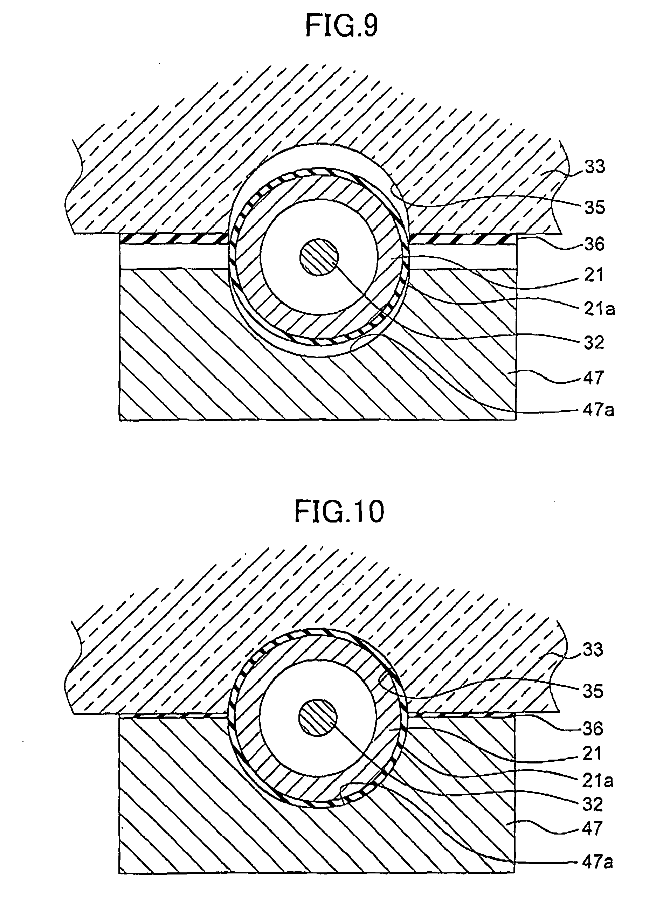

The cassette pressing member 33 has a groove 35, a seal member 36, a stepped portion 37, and a pressing elastic member 38. Of these, the groove 35 is formed by cutting off the end of the member 33 to have a semi-circular section (refer to Fig. 9). The seal member 36 is an elastic member arranged at the end. Further, the pressing elastic member 38 is a member, such as rubber foam, arranged on the lower surface of the stepped portion 37.

The portion-being-broken latch member 34 has an end on which a claw portion 39 is formed. This claw portion includes an latch groove 39a for engaging the protruded end of the arm-shaped protrusion 26 of the brushing brush cassette 20. As will be detailed, the claw portion 39 functions not only a member for pressing the washing brush cassette 20 but also a breaking member for positively breaking off part of the washing brush cassette 20. The claw portion 39 has the latch groove 39a formed to have a hook shape (refer to Fig. 6).

Moreover, the top cover 3 has a protruding end 40, as shown in Fig. 6. This protruding end 40 is formed to direct downward from the top cover 3 at a position close to the boundary of the cassette cover 3a on the washing bath side. A packing 41 is secured on the end of the protruding end 40.

The washing-nozzle connecting/disconnecting mechanism 45 will now be described in its configuration.

This connecting/disconnecting mechanism 45 is equipped with a casing 46, a cassette connecting portion 47, a washing nozzle 49, and a nozzle driving motor 53. Among them, the case member 46 is a recessed member to form part of the wall of the manipulating-member accommodating bath 6 of the washing bath 4. The cassette connecting portion 47 is integrally produced with the case member 46 and positioned to be farther than the case member 46, when viewing from the manipulating-member accommodating bath 6. The washing nozzle 49 is arranged in the casing 46 via the cassette connecting portion 47 so that the nozzle 49 is movable in the longitudinal direction thereof. The nozzle driving motor 53 is fixedly accommodated in the casing 46 and is equipped with a pinion gear 54 that makes the washing nozzle 49 go backward or forward (i.e., advance and retreat) in its longitudinal direction in a selective and controlled manner.

As shown in Fig. 6, the casing 46 has a wall portion of the manipulating-member accommodating bath 6 through which a hole is formed. A packing 48 is secured to the hole to gain an air (water)-tightness performance between the hole and the washing nozzle 49 passing through the hole.

The cassette connecting portion 47 is formed to have a hole portion 47a which communicates with the inner space of the case body 46. One end portion of the washing nozzle 49 is passed through the hole portion 47a. An upper end part of the cassette connecting portion 47, which is located far from the manipulating-member accommodating bath 6, is partly cut off. The cassette pressing member 33 extending from the cassette cover 3a is detachably engaged with this cut-ff part of the cassette connecting portion 47.

The washing nozzle 49 is an approximately cylindrical member and has a rack gear 49a on the outer circumferential surface of the nozzle 49. The washing nozzle 49 is arranged in the casing 46 such that the nozzle 49 is able to freely and linearly go back and forth through both of the hole with the packing 48 and the hole 47a of the cassette connecting portion 47.

The rack gear 49a on the washing nozzle 49 is disposed to be engaged with a pinion gear 54 of the nozzle driving motor 53 in the casing 46. Thus, the washing nozzle 49 is able to selectively and controllably advance and retreat toward and from the manipulating-member accommodating bath 6 in the washing bath 4 in reply to driving the motor 53.

On the outer surface of one end of the washing nozzle 49 (the rightward end illustrated in Figs. 5-8 and 11), an O-ring 49b is disposed to gain an air(water)-tightness performance between the hole portion 47a of the cassette connecting portion 47 and the nozzle 49. This washing nozzle 49 has a tip having a substantial cone shape on its leftward end, as shown in Figs. 5-8 and 11. The cone-like tip of the washing nozzle 49 is detachably connected with a mouthpiece (not shown) attached to an endoscopic channel (duct) and located at the manipulating member 51 of the endoscope 50 in response to advancing and retreating the nozzle 49. Such endoscopic channels are for example a duct through which therapeutic instruments are inserted and which also serves as a suction duct.

The cassette mounting portion 5 will now be described in terms of its configuration and operations.

As shown in Figs. 6-8 and 11, the cassette mounting portion 5 contains a mounting surface 55 on which the washing brush cassette 20 is detachably mounted. The cassette mounting portion 5 is located at one upper corner of the endoscope washing and disinfecting apparatus 1, as shown in Fig. 1.

In the cassette mounting portion 5 are disposed a brush driving motor 56 having a rotating shaft 57, a roller-shaft connecting portion 58, an elastic spring 59, two engaging holes 61 (only one hole is shown in Fig. 6), a touch senor 62, and a crank-shaped duct connecting/disconnecting pipe 64 in this order from the manipulating-member accommodating bath 6. Of these elements, the roller-shaft connecting portion 58 is coupled with the shaft 57 of the brush driving motor 56. The elastic spring 59 is an elastic member with a disk-like plate 60 secured atop the spring 59. The touch sensor 62 has a touch switch member 62a inserted in one or both of the two engaging holes 61, so that the touch sensor 62 serves as a touch sensing device. Further, the duct connecting/disconnecting pipe 64 is coupled, via a fluid supply tube 63, with a not-shown fluid supply duct supplying fluid circulating in the frame 2A. Such fluids include washing liquid, disinfecting liquid, alcohol, rinsing water and air.

The roller-shaft connecting portion 58 is sustained to be rotatable to the mounting surface 55. Not-shown sealing members are provided to gain an air-tight performance between the roller-shaft connecting portion 58 and the mounting surface 55. The roller-shaft connecting portion 58 has an engaging hole 58a, of which contour is the same as the roller shaft 22b with the engaging portion 22B of the washing brush cassette 20. The brush driving motor 56 is driven by a control unit 100 (refer to Fig. 6) in the main body unit 2 in a controlled manner.

Since the elastic spring 59 has one end secured on the bottom of the hole formed in the mounting surface 55, this spring 59 is able to generate an upward pushing force. Additionally, this spring 59 is designed in a manner that, when pressed down from the top thereof, the spring 59 is accommodated entirely in the hole and allows its plate 60 to cover the hole.

The touch sensor 62 has the touch switch member 62a, as described, and, when the touch switch member 62 is pressed down, generates an electric detection signal to be fed to the control unit 100 placed in the main body unit 2. The touch sensor 62 may be loaded into either the two engaging holes 61 or loaded into both engaging holes 61.

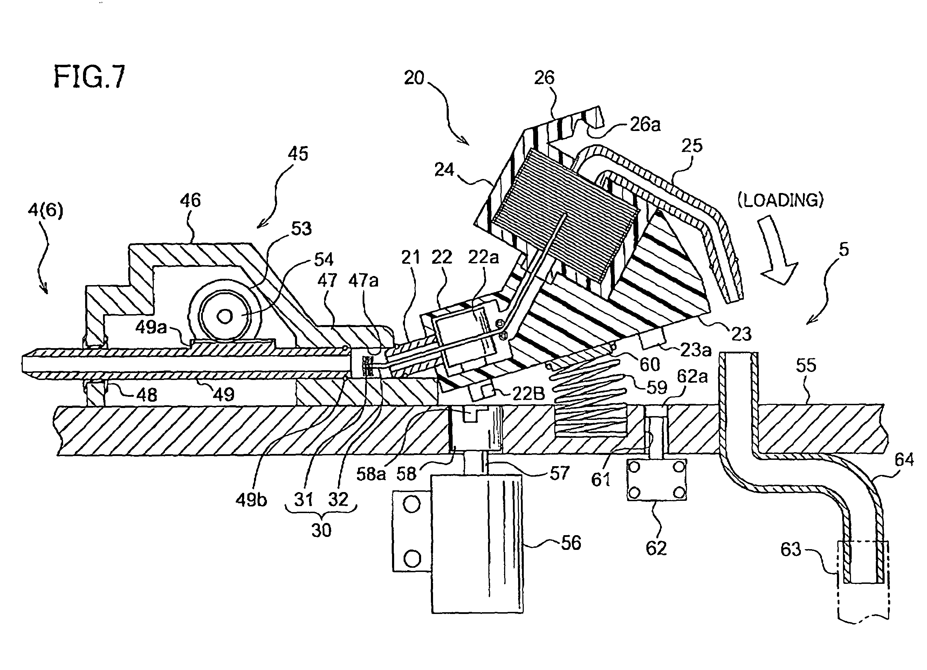

In the present embodiment, the endoscope washing and disinfecting apparatus 1 and washing brush cassette 20 are constructed as above. Hence, when using the apparatus 1, an operator first opens the top cover 4, and then, as illustrated in Fig. 7, the washing brush cassette 20 is mounted onto the cassette mounting portion 5.

For mounting the cassette 20, the operator is to locate the exhaust-side connecting pipe 21 at the cassette connecting portion 47 of the washing-nozzle connecting/disconnecting mechanism 55. Concurrently with this location, the user is to mount the cassette 20 such that the tip of the exhaust-side connecting pipe 21 of the cassette 20 engages with the cassette connecting portion 47. In this mounted state, the upward pushing force generated from the spring 59 makes it possible that the plate is made to touch the back surface of the base portion 23, pushing the washing brush cassette 20 upward.

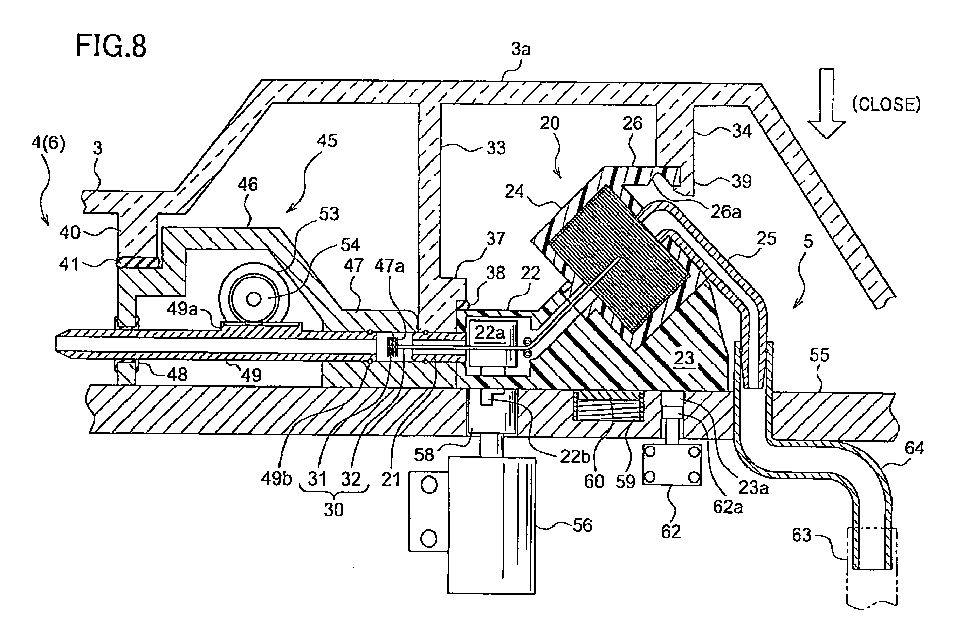

As the next step, the operator has an endoscope 50 accommodated in the washing bath 4, as shown in Fig. 1. After this accommodation, the top cover 3 is closed toward the frame 2A to tightly close the washing bath 4. During this closing operation, as shown in Fig. 8, the cassette pressing member 33 and portion-being-broken latch member 34 of the cassette cover 3a are made to forcibly touch the washing brush cassette 20. By this touch, the cassette 20 is obliged to be pressed down toward the cassette mounting portion 5 against the elastic spring 59.

The pressing-down process utilizing the touch will now be detailed. As shown in Figs. 9-10, the groove 35 of the cassette pressing member 33 is made to touch the exhaust-side connecting pipe 21 and, as shown in Fig. 8, the pressing elastic member 38 of the stepped portion 37 is made to touch the upper surface of the roller accommodating portion 22. Hence the washing brush cassette 20 is forced to be pressed down to the cassette mounting portion 5. During this pressing, the tip of the arm-shaped protrusion 26 of the cassette 20 is forcibly inserted into and latched with the latch groove 39a formed on the claw portion 39 of the portion-being-broken latch member 34. Thus, the portion-being-broken engaging member 34 enables the cassette 20 to be pressed toward the mounting portion 5 via the arm-shaped protrusion 26.

At this time, as to the washing brush cassette 20, the roller shaft 22b protruding from the roller accommodating portion 22 is tightly inserted into the engaging hole 58a of the roller-shaft connecting portion 58, each of the protrusions 23a protruding from the base portion 23 is tightly inserted into each of the holes with the touch sensors 62 accommodated therein, and the lower end of the suction-side connecting pipe 25 is tightly inserted into the duct connecting/disconnecting pipe 64, respectively. In addition, the plate 60 and the elastic spring 59 both are forcibly contained within the hole portion formed in the mounting surface 55.

When the closing operation of the top cover 3 onto the frame 2A is completed, the protrusions 23a of the washing brush cassette 20 press down the touch switch members 62a of the touch sensor 62. In reply to these pressing operations, the detection signals are outputted from the touch sensors 62 to the control unit 100 placed in the main body apparatus 2. Outputting the detection signals reveals that the washing brush cassette 20 has been mounted onto the cassette mounting portion 5 without fail.

With reference to Fig. 12, the processing executed by the control unit 100 (that is, a CPU (central processing unit) contained therein) will now be described. The control unit 100 tries to read in the electric detection signals that may come from the touch sensors 62 and determines whether or not the detection signals have been given actually (step S1). In a case where it is confirmed that the detection signals have been inputted (step S1, YES), the control unit 100 makes the operation/display panel 8 of the top cover 3 render the completion of setting for the washing and disinfection (step 52).

Then the control unit 100 tries to read in a start signal being outputted from the start switch placed in the operation/display panel 8 and determines whether or not the start signal has been inputted actually (step S3). This process will be repeated until the actual input of the start signal. On confirming the actual input of the start signal (step S3, YES), the control unit 100 proceeds to the next process where programs on predetermined washing and disinfecting procedures are executed to drive the various mechanisms and devices in the main body unit 2 (step S4). By this processing, the outer surface and ducts of the endoscope 50 are washed and disinfected.

Meanwhile, if it is determined that the process at step S1 is NO, that is, the detection signals have not been inputted yet, the control unit 100 proceeds to the next step to determine whether or not warning should be issued (step S5). This determination can be made based on, for example, an algorism defined such that "if there are no detection signals during a predetermined period of waiting time counted from a time instant when it was confirmed the closing action of the top cover 3, an alarm is issued." When the current situation does not meet such an algorithm, the control unit 100 returns its processing to step S1. In contrast, when it is determined that the alarm should be issued (step S5, YES), the alarm is given by illuminating an alarm lamp of the operation/display panel 8 or sounding a buzzer disposed of the panel 8 (step S6). After the alarming, the control unit 100 waits for operator's next command.

In this way, the control is carried out by the control unit 100. Therefore, in the endoscope washing and disinfecting apparatus 1 according to the present embodiment, the operation/display panel 8 of the top cover 3 is commanded to display the completion of setting for washing and disinfection. This display shows that the endoscope 50 is ready for the washing and disinfection.

In the present endoscope washing and disinfecting apparatus 1, the control unit 100 gives no control enabling the washing and disinfection to be started, as long as the detection signals are not received, even when the operator presses the start switch in the operation/display panel 8. Meanwhile, the control unit 100 executes the control to display the alarm lamp and/or sound the alarm buzzer in order to notify that the washing brush cassette 20 has been erroneously mounted.

The operator turns on the start button at the operation/display panel 8, after it has been notified that the washing brush cassette 20 has been mounted on the cassette mounting portion 5 without fail. Responsively, the endoscope washing and disinfecting apparatus 1 operates to comply with the predetermined procedures for the washing and disinfection, resulting in that the outer surface of the endoscope 50 is washed and disinfected and the endoscopic channel (in the present embodiment, the insertion duct for therapeutic instruments) of the endoscope 50 is brush-washed by the brush portion 31 that is made to advance and retreat along the channel.

In the brushing washing, the control unit 100 in the main body unit 2 operates to drive in a controlled manner both of the brush driving motor 56 and the nozzle driving motor 53. Specifically, first of all, the nozzle driving motor 53 is driven, so that the washing nozzle 49 is allowed to advance toward the duct mouthpiece attached to the manipulating member 51 of the endoscope 50, and to be connected to the duct mouthpiece. Then the brush driving motor 56 is driven to rotate alternately in both rotating directions, so that the shaft wire 32 pinched between the two rollers 22a goes forward and backward, alternately, thereby providing alternate advancing and retreating motions to the brush portion 31 within and along the duct for therapeutic instruments.

At this time, via the fluid supply tube 63 and the duct connecting/disconnecting pipe 64, the washing liquid and rinsing water, which circulate in the frame 2A, inflows into the washing brush cassette 20 from the suction-side connecting pipe 25. And, the washing liquid and the rinsing water pass the washing-brush accommodating portion 24, the communicating path 23b, the roller accommodating portion 22, and the exhaust-side connecting pipe 21, before reaching the washing nozzle 49. After passing the washing nozzle 49, the washing liquid and the rinsing water inflows the endoscopic duct for therapeutic instruments.

On completion of the brushing washing using the brush portion 31, the brush driving motor 56 is driven to cause the shaft wire 32 to be pulled back and accommodated into the washing-brush accommodating portion 24.

In the same way as the washing and rinsing steps, in a state where the brush portion 31 is stopped from going back and forth, the disinfecting liquid, alcohol, and air are supplied via the fluid supply tube 63 to the cassette mounting portion 5 and the washing brush cassette 20, and then to the therapeutic-instrument inserting duct of the endoscope 50.

Because the disinfecting liquid and rinsing water flows within the washing brush cassette 20, both the brush portion 31 and the shaft wire 32 are also disinfected and rinsed as well as the inside of the cassette 20. Hence it is possible to avoid that an operator gets dirty due to the disinfecting liquid, filth, and so on, when the operator removes the washing brush cassette 20 from the apparatus 1.

When the washing and disinfecting work is finished, the operator opens the top cover 3 to pick up the washed and disinfected endoscope 50 from the washing bath 4 of the endoscope washing and disinfecting apparatus 1.

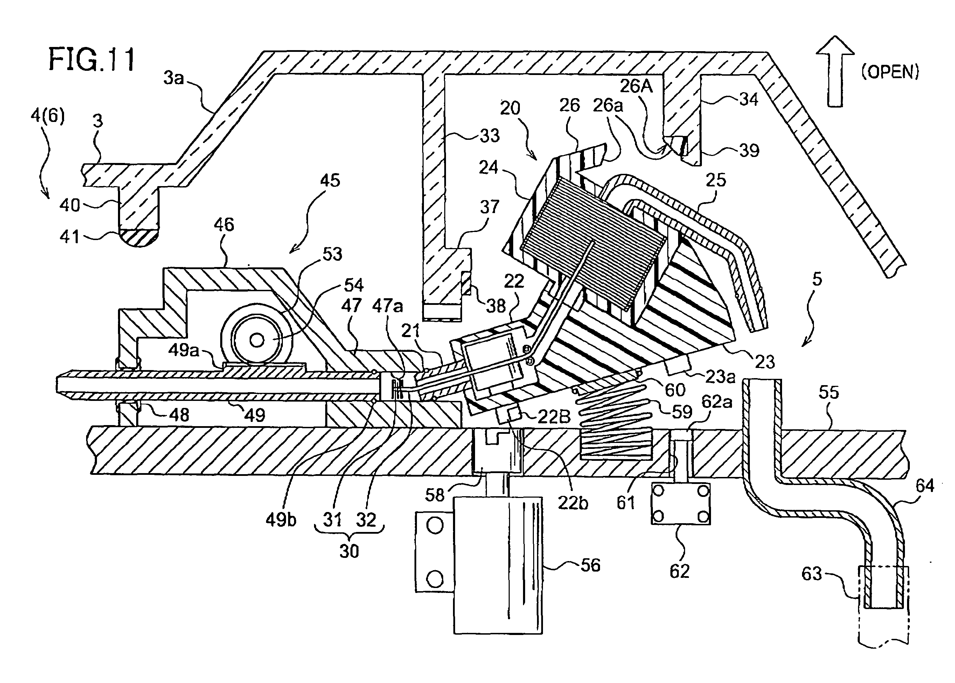

In this picking-up operation, as shown in Fig. 11, the operator moves the top cover 3 upward to be opened. This movement allows the elastic spring 59 to push the washing brush cassette 20 upward. And the pressing elastic member 38, arranged at the stepped portion 37 of the cassette pressing member 33, is released from pressing the roller accommodating portion 22.

Meanwhile, the arm-shaped protrusion 26 of the washing-brush accommodating portion 24 is obliged to move upward, while the tip 26A of the protrusion 26 is still latched with the latch groove 39a of the claw portion 39 of the portion-being-broken latch member 34 which is part of the cassette cover 3a. During this movement, the tip of the exhaust-side connecting pipe 21 catches on the tip of the cassette connecting portion 47, so that the washing brush cassette 20 is tilted. If there is no forcible moving-up force being applied to the cassette 20, it is no longer difficult for this cassette 20 to be moved upward any more from this caught-on position.

However, because the operator should open the top cover 3 to pickup the endoscope 50, the operator does so. Thus, the top cover 3, that is, cassette cover 3a, is forcibly moved (lifted) upward beyond the limit giving no damage to the arm-shaped protrusion 26. Thus, the opening action applied to the top cover 3 makes it possible to bend the arm-shaped protrusion 26 at its recessed notch 26a, which is the weakest point thereof. Therefore, at a positional point beyond the foregoing limit, the arm-shaped protrusion 26 is finally broken down at the notch 26a and separated into its main body and the tip 26A, as shown in Fig. 11.

Then, the operator opens fully the top cover 3, so that the washed and disinfected endoscope 50 can be taken out of the washing bath 4, before the washing and disinfecting work is ended. And the operator picks up the used washing brush cassette 20 from the cassette mounting portion 5 for putting it on the shelf. The pick-up cassette 20 is already broken down. This breakage is done positively (i.e., deliberately for avoiding an erroneous reuse). In this case, the arm-shaped protrusion 26 is disabled in its pressing and latching capabilities necessary for the cassette 20.

As described so far, the endoscope washing and disinfecting apparatus 1 equipped with the washing brush cassette 20 is provided. And the user is able to easily replace the washing brush cassette 20 by a new one. For achieving such an easier replacement, the washing brush cassette 20 is configured to be detachable to and from the apparatus 1. Hence it is possible to avoid work for replacing long washing brushes. In addition, the used washing brush cassette 20 can be detached with the long shaft wire 32 packed therein. The used cassette 20 can easily disposed of.

Furthermore, in the endoscope washing and disinfecting apparatus 1 according to the present embodiment, the arm-shaped protrusion 26 protruded from the washing brush cassette 20 is positively (deliberately) broken down. It is therefore possible to prevent the reuse of the washing brush 30 of which washing performance is poor due to the previous use.

Namely, once the washing brush cassette 20 is used, its arm-shaped protrusion 26 is broken so that the cassette 20 is disabled from being reused. An operator is able to visually confirm the broken protrusion 26 and to easily determine whether a washing brush cassette 20 is already used or not.

In addition, if an operator loads a used washing brush cassette 20 to the apparatus 1 by mistake, it is impossible to securely mount the cassette 20 onto the cassette mounting portion 5 when the top cover 3 is closed. The reason is that, owing to the fact that the tip 26A is already lost, the distal end of the protrusion 26 cannot be latched with the latch groove 39a of the portion-being-broken latch member 34, and the cassette 20 is pushed up by the elastic spring 59 so that the mounting action is disabled.

Additionally, the pushed-up cassette 20 prevents the protrusions 23a protruded from the base portion 23 from reaching the touch switch members 62a of the touch sensor 23a, whereby the touch switch members 62a cannot be pressed down. In this situation, thanks to control executed by the control unit 100 which has sensed an erroneous mounting state of the cassette 20, an operator's action of pressing the start switch of the operation/display panel 8 will be disabled from staring the washing and disinfection. Instead, the control unit 100 executes control to issue an alarm through the buzzer, for example. Such an alarm will notifies an operator of the current situation where the operator now intends to use the previous used cassette 20 again by mistake.

The same merit is also true of a situation where, with the used washing brush cassette 20 left at the cassette mounting portion 5 without a replacement thereof, an operator closes the top cover 3 to repeatedly wash and disinfect another endoscope 50. In this case, the used cassette 20 cannot be mounted tightly at the mounting portion 5, thus being disabled from staring the washing and disinfecting operations.

As described, in the endoscope washing and disinfecting apparatus 1 and the washing brush cassette 20 according to the present embodiment, the endoscopic channels (ducts) of a used endoscope 50 can be washed and disinfected in a sanitary and effective fashion. The washing brush 30 with a degraded washing capability, due to the previous usage, can easily and quickly be replaced by a new unused one. Further, it is reliably prevented that such used washing brushes fail to be replaced and are used by mistake.

Various modifications can still be applied to the configurations exemplified in the above embodiment. For example, the embodiment has been descried about the endoscopic channel (duct) for therapeutic instruments which is usually subjected to heavy pollution. However, endoscopic channels to which the present invention is applied are not always limited to such a channel, but any endoscopic channel can be brushing-washed on the present invention.

Further, in the foregoing embodiment, the washing brush cassette 20 has been explained as a disposal type of bush unit which is disposed of, every use. And, for making it sure that the cassette 20 is used only one time, the arm-shaped protrusion 26 is formed on the cassette 20. This protrusion 26 operates cooperatively with the claw portion 39 of the portion-being-broken latch member 34, so that the cassette 20 is pressed downward for the fixed mounting thereof as well as disabled (stopped) from being reused thanks to the positive breakage of the tip 26A of the protrusion 26. In this regard, the protrusion 26 and the claw portion 39 can further be modified into various other modes.

For instance, the protrusion 26 may not be shaped into an arm. Any shape of protruded portion may be adopted. The tip 26A of the protrusion 26 may not always be formed to be divided by the recessed notch (groove) 26a. Another example is shown in Fig. 13. As shown therein, the tip may be formed to be just deformed, not completely broken down. The notch 26a may be replaced by a though-hole laterally passing through the protrusion 26 at a proper position in the thickness direction thereof. The notch 26a is a representative of members or structures for assisting the positive breakage or deformation of part of the cassette 20. Thus it is sufficient for the notch 26a that the tip 26A bears the pushing-down force without breakage and any deformation and is always fragile to the pushing-up force, with breakage or deformation caused.

The shape of the latch groove 39a formed on the claw portion 39 is also not necessarily limited to a hook shape or a key shape. As long as the pushing-down and pushing-up forces applied when the top cover 3 is closed and opened respectively can be transformed to the protrusion 26, any shape is applicable to the latch groove 39a.

Although the description above contains many specificities, these should not be construed as limiting the scope of the invention but as merely providing illustrations of some of the presently preferred embodiments of the present invention. Thus the scope of the present invention should be determined by the appended claims.

## Claims

1. An endoscope washing and disinfecting apparatus (1) for washing and disinfecting an endoscope (50) having a duct, the apparatus comprising:
a frame (2A) equipped with a washing bath (4) accommodating therein the endoscope for washing and disinfection;
a brush unit (20) accommodating therein a brush (30) for washing the duct of the endoscope and detachably loaded to the frame at a predetermined position thereof to allow the brush to be inserted into the duct;
a lid (3) attached to the frame to selectively open and close and formed to cover the washing bath and the brush unit loaded to the fame when the frame is closed; and
a reuse-disabling device (34, 39) disabling the brush unit from being reused in association with the opening and closing actions of the lid.

2. The endoscope washing and disinfecting apparatus of claim 1, wherein the reuse-disabling device is disposed on the lid.

3. The endoscope washing and disinfecting apparatus of claim 2, wherein the reuse-disabling device is formed as a breaking-down member (39) positively breaking down part (26) of the bush unit to disable the brush unit from being re-loaded to the frame.

4. The endoscope washing and disinfecting apparatus of claim 3, further comprising a securing member (34) being arranged on the lid and pressing the brush unit toward the frame to secure the brush unit when the lid is closed to the frame.

5. The endoscope washing and disinfecting apparatus of claim 4, wherein the breaking-down member is disposed on the securing member.

6. The endoscope washing and disinfecting apparatus of claim 5, wherein the part of the brush unit is a protrusion made to touch the securing member to be pressed and made to be latched with the breaking-down member, when the lid is closed to the frame.

7. The endoscope washing and disinfecting apparatus of claim 6, wherein
the breaking-down member has a hook structure (39a) formed at a tip of the protrusion and
the protrusion comprises facilitating means (26a) for facilitating the positive breakage using a force applied to a tip (26A) of the protrusion by the breaking-down member, the force being generated when the lid is opened.

8. The endoscope washing and disinfecting apparatus of claim 7, wherein
the protrusion is an arm-shaped member having the tip and the facilitating member is formed by a notch (26a) formed in the arm-shaped member.

9. The endoscope washing and disinfecting apparatus of claim 4, wherein the
the reuse-disabling device is configured to disable the securing member for pressing the brush unit toward the frame, when the brush unit is reloaded to the frame.

10. The endoscope washing and disinfecting apparatus of claim 1, wherein the
the reuse-disabling device is configured to positively break down or deform part (26) of the brush unit such that the brush unit is disabled from being reloaded to the frame.

11. The endoscope washing and disinfecting apparatus of claim 1, further comprising
plural securing members (33, 34) being arranged on the lid and pressing the brush unit toward the frame to secure the brush unit, when the lid is closed to the frame, and
a detector (23a, 62) detecting whether or not the brush unit is normally secured at the predetermined position of the frame, when the lid is closed on the frame.

12. The endoscope washing and disinfecting apparatus of claim 11, wherein the reuse-disabling device is disposed on the lid.

13. The endoscope washing and disinfecting apparatus of claim 12, wherein the reuse-disabling device is formed as a breaking-down member (39) positively breaking down part (26) of the bush unit to disable the brush unit from being re-loaded to the frame.

14. The endoscope washing and disinfecting apparatus of claim 13, wherein the breaking-down member is disposed on one (34) of the securing members (33, 34).

15. The endoscope washing and disinfecting apparatus of claim 11, further comprising an alarming member (100, 8) issuing an alarm indicating that the brush unit is not normally secured on the frame, when the detector detects that the brush unit is not normally secured on the frame.

16. The endoscope washing and disinfecting apparatus of claim 11, wherein the brush unit comprises
a pipe-shaped cylindrical member (21) through which the brush is fed toward the duct of the endoscope and
a sealing member loaded on an outer surface of the cylindrical member and sealing a gap between (i) the cylindrical member pinched by part of the frame and part (34) of the securing members and (ii) the parts of both the securing members and the frame, when the brush unit is loaded on the frame.

17. A brush unit comprising:
a brushing device (30) equipped with a brush (31) washing a duct of an endoscope;
a drive member (22a, 22b) driving the brushing device to run the brush in and along the duct of the endoscope;
a casing (24) accommodating therein the brushing device and the drive member and being detachably loaded to a washing and disinfecting apparatus for washing and disinfecting the endoscope; and
a reuse-stopping member (26) being arranged at the casing and stopping the brush unit from being reused.

18. The brush unit of claim 17, wherein the reuse-stopping member comprise a portion (26A) being broken positively when a lid of the washing and disinfecting apparatus is opened, the positive breakage disabling the brush unit from reloaded to the washing and disinfecting apparatus.

19. The brush unit of claim 18, wherein the reuse-stopping member includes a notch (26a) facilitating the breakage of the portion being broken, using a force given to the portion being broken, when the lid is opened.
